# EUROPEAN PATENT APPLICATION

(11) **EP 3 195 790 A1**
(43) Date of publication of application: **26.07.2017**
(21) Application number: 16807179.3
(22) Date of filing: 23.03.2016
(51) Int. Cl.: A61B 1/04, G02B 23/24

(54) **SIGNAL LINE INSTALLATION STRUCTURE OF ELONGATED MEMBER**

(30) Priority: 10.06.2015 JP 2015117714
(71) Applicant: Olympus Corporation, Tokyo 192-8507 (JP)
(72) Inventor: SEKIGUCHI, Masahiko, Hachioji-shi Tokyo 192-8507 (JP)
(74) Representative: Schicker, Silvia
(86) International application number: PCT/JP2016/059152
(87) International publication number: WO 2016/199472

(57) **Abstract**

A signal line installation structure of an elongated member includes a signal line 6 that is installed between an inner circumferential surface of an outer skin tube 2 and outer circumferential portions of a rigid portion 4 and a flexible portion 5, where the signal line 6 is installed according to an installation structure according to which the signal line 6 includes a spiral portion 6b that is helically wound around the rigid portion 4 and the flexible portion 5 with an electrical connection portion 6a on the outer circumference of the rigid portion 4 as a winding start point. The spiral portion 6b may prevent disconnection by reducing the bending stress of tension and compression that is applied to the signal line 6 itself when the flexible portion 5 is bent.

## Description

### Technical Field

The present invention relates to a signal line installation structure of an elongated member which is capable of preventing disconnection of a signal line caused by bending.

### Background Art

Generally, an elongated member such as an insertion section of an endoscope to be inserted into a subject includes inside a rigid portion having rigidity and a flexible portion having flexibility. The rigid portion and the flexible portion are covered by an outer skin tube, and a signal line is installed according to a predetermined installation structure, between the outer circumferential surfaces of the rigid portion and the flexible portion and the inner circumferential surface of the outer skin tube.

For example, with respect to such an installation structure of a signal line, Japanese Patent Application Laid-Open Publication No. 2001-75022 discloses a structure according to which a helical groove is formed on the outside of a treatment instrument channel (a flexible tube) inserted in an insertion section of an endoscope, a signal line is wound in the helical groove from the hand side, and the distal end side of the signal line is connected to an image pickup unit disposed at a side of the treatment instrument insertion channel.

However, Japanese Patent Application Laid-Open Publication No. 2001-75022 simply discloses an installation structure of a signal line, the object of which is to reduce the diameter of the insertion section, and does not particularly take into consideration a bending stress that is applied to a wire portion, of the signal line, from a rigid portion to a flexible portion on the inside.

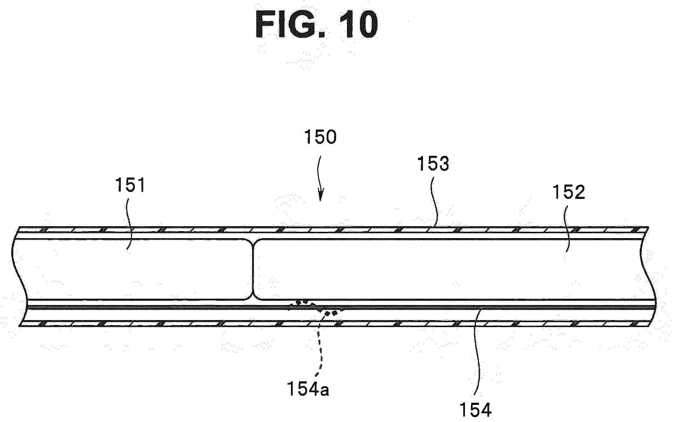

For example, as shown in Fig. 10, an elongated member 150 includes inside a rigid body 151 having rigidity and a flexible body 152 having flexibility that are disposed next to each other, and a signal line 154 is linearly disposed between an outer skin tube 153 covering the rigid body 151 and the flexible body 152, and when the elongated member 150 is repeatedly bent, a bending stress is repeatedly applied to the signal line 154, possibly leading to disconnection.

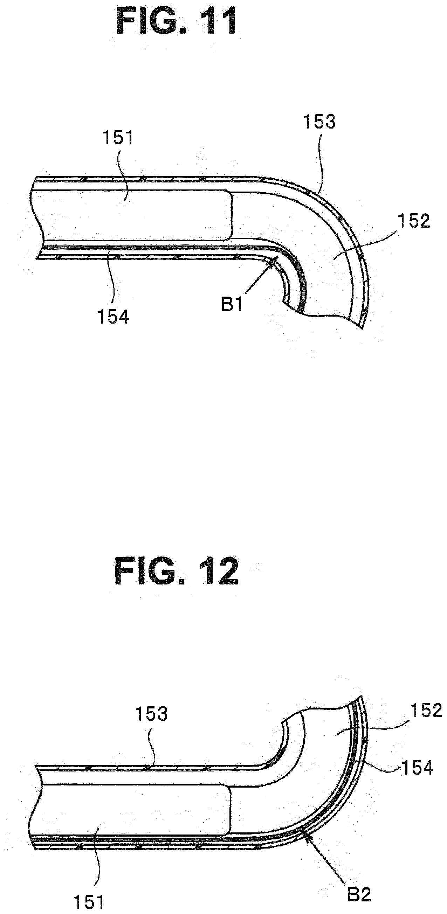

That is, when the elongated member 150 is bent, the flexible body 152 is bent from an adjacent portion between the rigid body 151 and the flexible body 152 inside the elongated member 150, and when the signal line 154 is on the inner side of the bending, as shown in Fig. 11, a force in the compression direction is applied to a B1 portion of the signal line 154, and when the signal line 154 is on the outer side of the bending, as shown in Fig. 12, a force in the tensile direction is applied to a B2 portion of the signal line 154. When such compression/tensile forces are repeatedly applied to the signal line 154, the signal line 154 is partially slackened, as shown by a broken line in Fig. 10, and a bent warped portion 154a will appear, possibly leading to disconnection.

The present invention has been made in view of the above circumstances, and has its object to provide a signal line installation structure of an elongated member which is capable of preventing disconnection of a signal line that is disposed inside even when the elongated member is repeatedly bent.

### Disclosure of Invention

### Means for Solving the Problem

A signal line installation structure of an elongated member according to an aspect of the present invention is a signal line installation structure of an elongated member including an outer skin tube that is cylindrically formed and that extends along a predetermined axis, an internal component that is housed inside the outer skin tube and that includes a rigid portion and a flexible portion that are disposed next to each other in a direction of the axis, and a signal line that is installed across the rigid portion and the flexible portion and between an inner circumferential surface of the outer skin tube and an outer circumferential surface of the internal component, where the signal line that is disposed at outer circumferential portions of the rigid portion and the flexible portion is at least partially helically wound around outer circumferential surfaces of the rigid portion and the flexible portion.

### Brief Description of the Drawings

Fig. 1 is a basic configuration diagram showing a signal line installation structure according to the present invention;
Fig. 2 is an explanatory diagram showing a winding start point of a signal line;
Fig. 3 is an explanatory diagram showing a load that is applied to a spiral portion;
Fig. 4 is an explanatory diagram showing a signal line installation structure where the spiral portion has a different winding start point;
Fig. 5 is an explanatory diagram showing a load that is applied to the spiral portion in Fig. 4;
Fig. 6 is an explanatory diagram of an endoscope having the signal line installation structure according to the present invention;
Fig. 7 is a cross-sectional view showing an overview of a distal end portion of an insertion section of the endoscope;
Fig. 8 is a cross-sectional view of an image pickup unit;
Fig. 9 is a cross-sectional view of a driving force generation portion and a coupling portion;
Fig. 10 is an explanatory diagram showing a conventional signal line installation structure;
Fig. 11 is an explanatory diagram showing generation of a compression force according to the signal line installation structure in Fig. 10; and
Fig. 12 is an explanatory diagram showing generation of a tensile force according to the signal line installation structure in Fig. 10.

### Best Mode for Carrying Out the Invention

Hereinafter, an embodiment of the present invention will be described with reference to the drawings. Note that, in each of the drawings used in the following description, the scale of display of each structural component is made different such that each structural component is large enough to be recognized in the drawing. Accordingly, the present invention is not limited to the modes shown in the drawings with respect to the number of structural components, the shapes of the structural components, the proportion of the sizes of the structural components, and the relative positional relationship of respective structural components.

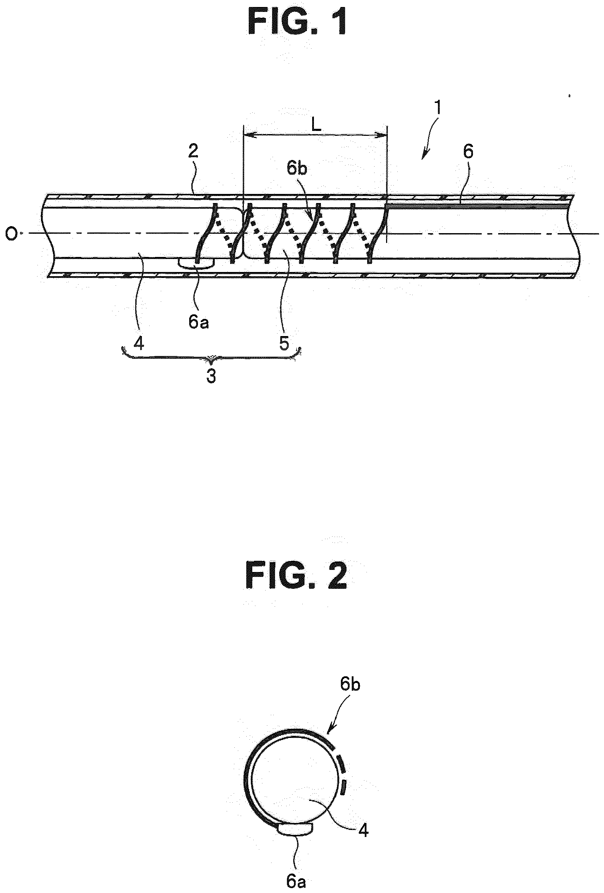

First, a basic configuration of a signal line installation structure of the present invention will be described with reference to Fig. 1. An elongated member 1 having the signal line installation structure of the present invention includes an outer skin tube 2 that is cylindrically formed and that extends along a predetermined axis O, an internal component 3 housed inside the outer skin tube 2, and a signal line 6 that is installed according to an installation structure described below, between an inner circumferential surface of the outer skin tube 2 and an outer circumferential surface of the internal component 3. The elongated member 1 including such a signal line 6 is applied as a part of a drive mechanism for changing the image pickup magnification of an image pickup unit of an endoscope described below, for example.

The outer skin tube 2 is a pipe, of resin or the like, which covers the internal component 3 and the signal line 6, and which has flexibility so as to be bendable according to bending of the elongated member 1. Moreover, the internal component 3 is a structural body including a rigid portion 4 and a flexible portion 5 that are disposed next to each other in the direction of the axis O. The rigid portion 4 is formed of a rigid member having resistance against a force in the bending direction, and the flexible portion 5 is formed of a flexible member having flexibility allowing bending according to a force in the bending direction.

In the present embodiment, the rigid portion 4 is formed of a metal material so as to allow electrical connection with the signal line 6, and a distal end of the signal line 6 is soldered to a predetermined position on the outer circumference of the metal rigid portion 4, and an electrical connection portion 6a is thus formed. For its part, the flexible portion 5 is formed by a single member or by a combination of a plurality of members, and is configured to have flexibility as a whole.

The signal line 6 is installed between the inner circumferential surface of the outer skin tube 2 and the outer circumferential portion extending across the rigid portion 4 and the flexible portion 5. The signal line 6 is disposed according to an installation structure according to which at least a part of the signal line 6 disposed at the outer circumferential part of the rigid portion 4 and the flexible portion 5 is helically wound around the outer circumferential surfaces of the rigid portion 4 and the flexible portion 5.

More specifically, the signal line 6 includes a spiral portion 6b which is helically wound around the rigid portion 4 and the flexible portion 5 with the electrical connection portion 6a to the rigid portion 4 as the winding start point. That is, as shown in Fig. 2, the signal line 6 helically extends along the outer circumferential surface of the rigid portion 4 from the electrical connection portion 6a on the outer circumference of the rigid portion 4, and the spiral portion 6b is provided so as to be wound from around the outer circumferential surface of the rigid portion 4 and the outer circumferential surface of the flexible portion 5.

The pitch (the winding gap) and the length (the length from the winding start point to the winding end point) of the spiral portion 6b are set taking into account the degree of bending of the flexible portion 5 in actual use, and is set to certainly include an adjacent portion between the rigid portion 4 and the flexible portion 5 and to cover a part of the flexible portion 5 which is near the rigid portion 4 and which is to be bent. For example, a length L of the spiral portion 6b from an end portion of the rigid portion 4 is set to be equal to or greater than the outside arc of the maximum bending diameter that is expected. The spiral portion 6b may be wound until a rear end connection portion not shown. The signal line 6 may be linearly disposed in a normal manner on a rear side with respect to the spiral portion 6b.

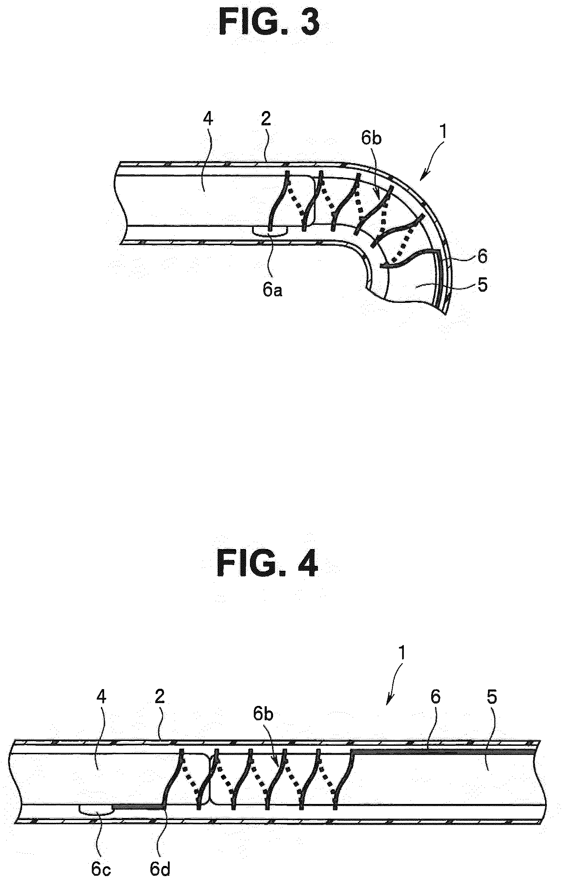

By adopting such an installation structure of the signal line 6, even if bending displacement is repeatedly caused near a fixed end of the signal line 6, the load applied to the signal line 6 may be reduced and disconnection may be prevented. That is, as shown in Fig. 3, when a force in the bending direction is applied to the elongated member 1, and bending displacement is caused at the flexible portion 5 with the surrounding of the adjacent portion of the rigid portion 4 and the flexible portion 5 as the origin, a stress in the tensile direction at an outside portion of bending and a stress in the compression direction at an inside portion of bending are converted into a force in a twisting direction at an obtuse angle at the spiral portion 6b formed extending over the flexible portion 5, with respect to the signal line 6 having the electrical connection portion 6a on the outer circumference of the rigid portion 4 as the fixed end, and the force in the twisting direction is applied to the spiral portion 6b.

The force in the twisting direction applied to the spiral portion 6b acts to change the winding pitch of the signal line 6, and the bending stress of tension and compression applied to the signal line 6 itself is greatly reduced. Accordingly, even if the flexible portion 5 is repeatedly bent, disconnection of the signal line 6 due to repeated bending stress may be reliably prevented.

Note that, as shown in Fig. 4, the winding start point of the spiral portion 6b of the signal line 6 does not necessarily have to be the electrical connection portion 6a of the signal line 6, and helical winding may be started by moving the position of the electrical connection portion 6a to a forward side (in a direction away from the flexible portion 5) and via a linearly drawn out portion, under a condition that the spiral portion 6b includes the adjacent portion between the rigid portion 4 and the flexible portion 5.

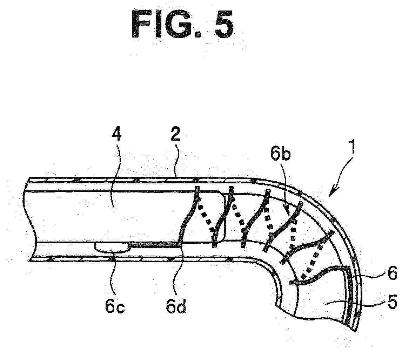

That is, an electrical connection portion 6c may be provided, as shown in Fig. 4, on the forward side with respect to the electrical connection portion 6a shown in Fig. 1, and the spiral portion 6b may be formed by helically winding the signal line 6 from a winding start position 6d linearly drawn out from the electrical connection portion 6c by a predetermined length along the outer circumference of the rigid portion 4. As shown in Fig. 5, also in such a case, a force that is applied to the spiral portion 6b when the flexible portion 5 is bent turns into a force in the twisting direction, and a tensile stress or a compression stress does not act on the signal line 6 itself, and disconnection of the signal line 6 may be reliably prevented.

However, compared to a case as shown in Fig. 1 where the position of winding start of the spiral portion 6b is at the electrical connection portion 6a, in the case where winding is started from the winding start position 6d shown in Fig. 4, the signal line 6 is helically bent at the position 6d from a straight state, and thus, a bending force may be applied to an end portion of the signal line 6 at the electrical connection portion 6c at the time of winding.

Also, for the spiral portion 6b to include the adjacent portion between the rigid portion 4 and the flexible portion 5, the winding start point of the spiral portion 6b has to be at an outer circumferential portion of the rigid portion 4, and management of the helix start position becomes important. If winding is started directly from the electrical connection portion 6a as shown in Fig. 1, variance in the helix start position of the signal line 6 at the time of assembly can be reduced further in the case of winding from the winding start position 6d slightly away from the electrical connection portion 6c as shown in Fig. 4, and stable quality can be obtained.

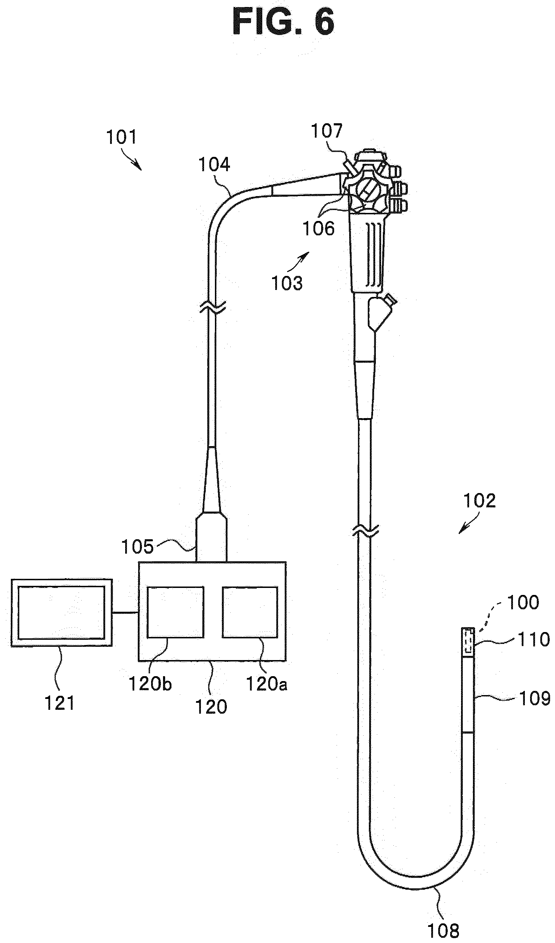

Next, as an application example of the signal line installation structure described above, a drive mechanism for changing the image pickup magnification of an image pickup unit of an endoscope will be described with reference to Figs. 6 to 9.

An endoscope 101 shown in Fig. 6 is configured to be insertable into a subject, such as a human body, and to optically pick up an image of a predetermined observation portion in the subject. The endoscope 101 is mainly configured from an insertion section 102 to be introduced into a subject, an operation section 103 positioned on a proximal end of the insertion section 102, and a universal cord 104 extending from a side portion of the operation section 103.

The insertion section 102 is configured by a distal end portion 110 disposed on a distal end, a bendable bending portion 109 disposed on the proximal end side of the distal end portion 110, and a flexible tube portion 108, having flexibility, disposed on the proximal end side of the bending portion 109 and connected on the distal end side of the operation section 103, the portions being disposed in a continuous manner.

Note that subject into which the endoscope 101 is to be introduced is not limited to a human body, and may be another living body, or an artificial object such as a machine or a building.

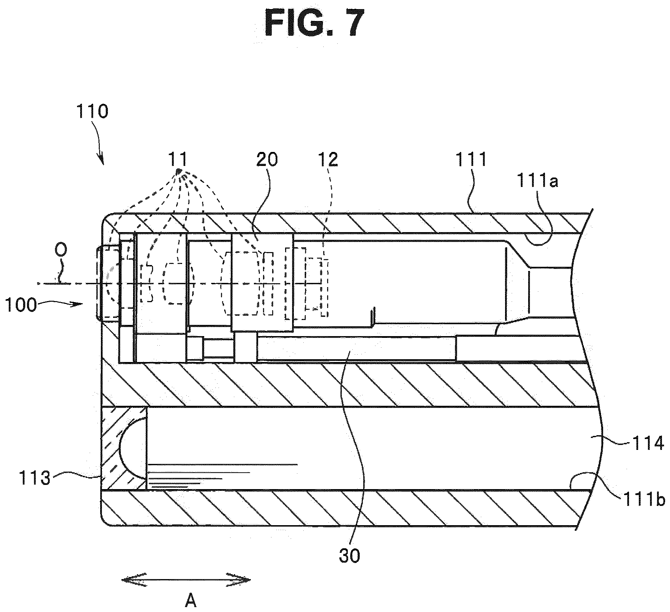

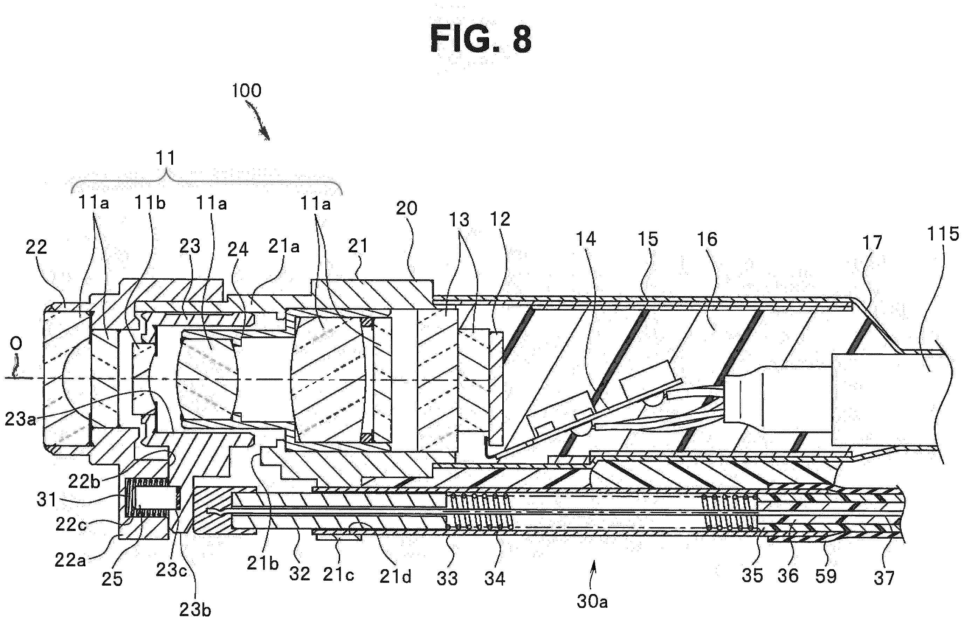

The operation section 103 is provided with an angle operation knob 106 for performing operation regarding bending of the bending portion 109. Also, a magnification operation section 107 is disposed at the operation section 103. The magnification operation section 107 is a lever switch for issuing instructions regarding operation of a drive mechanism section 30 described below (see Figs. 7 and 8) for changing the image pickup magnification of an image pickup unit 100, for example.

Furthermore, an endoscope connector 105 is provided at a proximal end portion of the universal cord 104. An electrical cable 115 and an optical fiber bundle 114 (see Figs. 7 and 8) inserted through the universal cord 104, the operation section 103, and the insertion section 102 are connected to the endoscope connector 105, and supply of illumination light from an external device 120 to the endoscope 101, supply of power to the image pickup unit 100, and communication between the external device 120 and the image pickup unit 100 are performed by connecting the endoscope 101 to the external device 120 via the endoscope connector 105.

For example, the external device 120 is configured by including a light source section, a power source section 120a, an image processing section 120b, and an image display section 121. Note that the light source section may be disposed in the operation section 103 or the distal end portion 110 of the endoscope 101.

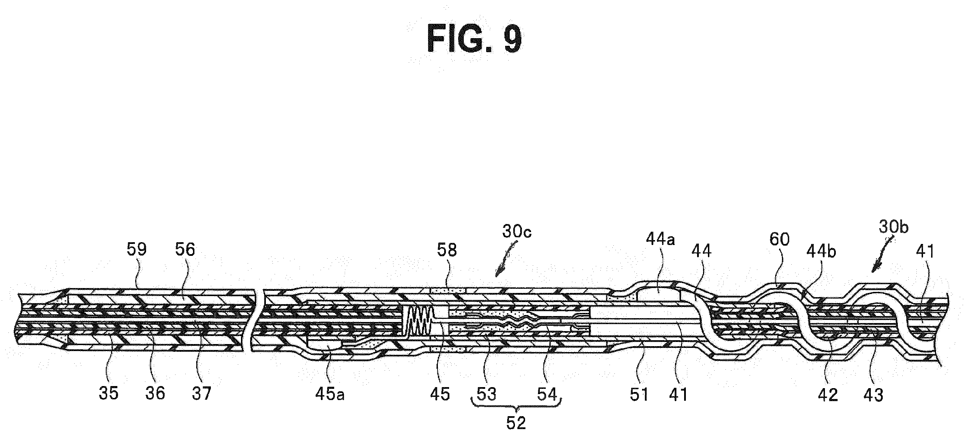

The power source section 120a is configured to output power for operating the drive mechanism section 30 provided to the image pickup unit 100, according to operation of the magnification operation section 107 by a user. As will be described in detail below, in the present embodiment, the power source section 120a is configured to apply an electrical current to a shape memory alloy wire (hereinafter abbreviated as "SMA wire"; see Fig. 9) 41, which is a shape memory alloy in the form of a wire, provided to the drive mechanism section 30.

The image processing section 120b is configured to generate a video signal based on an image pickup device output signal outputted from the image pickup unit 100, and to output the video signal to the image display section 121. That is, an optical image picked up by the image pickup unit 100 is displayed as a video by the image display section 121.

Next, a configuration of the distal end portion 110 will be described. As shown in Fig. 7, the image pickup unit 100, and an illumination light emitting section 113 for emitting illumination light transmitted through the optical fiber bundle 114 are disposed in the distal end portion 110.

In the present embodiment, the image pickup unit 100 is disposed to pick up an image in the distal end direction along the longitudinal direction (an insertion axis direction) of the insertion section 102 indicated by an arrow A in Fig. 7, for example. More specifically, the image pickup unit 100 is disposed such that an optical axis O of an objective lens 11 is along the longitudinal direction of the insertion section 102. Note that the image pickup unit 100 may be disposed in such a manner that the optical axis O forms a predetermined angle with the longitudinal direction of the insertion section 102.

Also, the illumination light emitting section 113 includes a component for emitting light transmitted from the light source section of the external device 120 through the optical fiber bundle 114 so as to illuminate an object of the image pickup unit 100. In the present embodiment, the illumination light emitting section 113 is configured to emit light along the longitudinal direction of the insertion section 102, in the distal end direction from the distal end surface of the distal end portion 110.

The image pickup unit 100 and the illumination light emitting section 113 are held by a holding section 111 provided to the distal end portion 110. The holding section 111 is a rigid member exposed from the distal end surface of the distal end portion 110, and through holes 111a and 111b are provided piercing the holding section 111 along the longitudinal direction of the insertion section 102. The image pickup unit 100 and the illumination light emitting section 113 are fixed inside the through holes 111a and 111b by means of adhesives or screws, for example. Moreover, the optical fiber bundle 114 is inserted and fixed inside the through hole 111b from the proximal end side.

Next, a configuration of the image pickup unit 100 of the present embodiment will be described. As shown in Figs. 7 and 8, the image pickup unit 100 is configured by including a lens barrel section 20 holding the objective lens 11 and an image pickup device 12 disposed on the image side of the objective lens 11, and the drive mechanism section 30 disposed at a side portion of the lens barrel section 20.

The objective lens 11 is formed from optical members, such as a plurality of lenses, for forming an object image. The objective lens 11 of the present embodiment is configured by including a fixed lens 11a, which is one or a plurality of lenses, with a fixed position in the lens barrel section 20, and a movable lens 11b, which is one or a plurality of lenses, capable of moving in the direction of the optical axis O in the lens barrel section 20.

The image pickup device 12 is an array of a plurality of light receiving elements for photoelectrically converting entering light, and generally, image pickup devices in the form of a CCD (charge coupled device) or a CMOS(complementary metal oxide semiconductor) sensor, or in various other forms may be adopted. The image pickup device 12 is disposed in such a way that the light receiving elements are arranged at the image forming surface of the objective lens 11.

A cover glass 13 is attached by an adhesive on the light receiving surface of the image pickup device 12 where the light receiving elements are arranged. Also, a circuit board 14 is electrically connected to the image pickup device 12. The circuit board 14 is electrically connected to the electrical cable 115.

The lens barrel section 20 holding the objective lens 11 and the image pickup device 12 described above is configured by including a fixed frame 21, an object-side lens holding frame 22, a movable lens holding frame 23, and an image-side lens holding frame 24. The fixed frame 21, the object-side lens holding frame 22, and the image-side lens holding frame 24 are substantially cylindrical members, and the positions are fixed with respect to one another by adhesives or by press fitting, for example.

The cover glass 13 is fixed on the proximal end side of the fixed frame 21 by an adhesive. That is, the image pickup device 12 is fixed to the proximal end side of the fixed frame 21 through the cover glass 13. On the other hand, a circular cylindrical portion 21a having a substantially circular cylindrical shape is provided on the distal end side of the fixed frame 21. A slit 21b, which is a through hole through which an arm portion 23b of the movable lens holding frame 23 described below is inserted, is formed at a side surface portion of the circular cylindrical portion 21a. The slit 21b is a long hole which takes a direction that is substantially parallel to the optical axis O as the longitudinal direction.

Furthermore, a holding portion 21c protruding in the radially outward direction (direction orthogonal to the optical axis O) in an arm-like manner is provided on the proximal end side, with respect to the slit 21b, on the side surface of the fixed frame 21. When seen from the direction of the optical axis O, the slit 21b and the holding portion 21c are provided in substantially the same circumferential direction with respect to the optical axis O. As will be described in detail below, the holding portion 21c is a part for positioning and holding a distal end portion of a guide pipe 33 constituting the drive mechanism section 30.

Specifically, a through hole 21 d, which is substantially parallel to the optical axis O, is formed at the holding portion 21c, and the guide pipe 33 having a substantially circular cylindrical shape is inserted and fixed in the through hole 21 d. As will be described in detail below, a pressing portion 32 is disposed in the guide pipe 33 in a manner capable of moving forward and backward in the direction of the optical axis O.

The object-side lens holding frame 22 is fixed at the distal end side of the circular cylindrical portion 21a of the fixed frame 21. The object-side lens holding frame 22 is a substantially circular cylindrical member for holding the fixed lens 11a, of the objective lens 11, positioned on the object side with respect to the movable lens 11b is.

A pressing protrusion portion 22a protruding in the radially outward direction (direction orthogonal to the optical axis O) in an arm-like manner is provided on the side surface of the object-side lens holding frame 22. When seen from the direction of the optical axis O, the pressing protrusion portion 22a is provided in substantially the same circumferential direction as the slit 21b with respect to the optical axis O. As will be described in detail below, the pressing protrusion portion 22a is a part constituting a part of the drive mechanism section 30. A flat portion 22b, which is substantially orthogonal to the optical axis O, is formed on the proximal end side of the pressing protrusion portion 22a.

A substantially circular recessed portion 22c recessed in the direction of the optical axis O is provided to the flat portion 22b. When seen from the direction of the optical axis O, the recessed portion 22c is disposed with a center position shifted from the through hole 21 d provided to the holding portion 21c of the fixed frame 21. Specifically, when seen from the direction of the optical axis O, the recessed portion 22c is disposed radially inward with respect to the through hole 21d of the fixed frame 21.

The movable lens holding frame 23 is disposed in the circular cylindrical portion 21a of the fixed frame 21 in a manner capable of moving forward and backward in the direction of the optical axis O. The movable lens holding frame 23 is a member for holding the movable lens 11b, of the objective lens 11, and is configured by including a lens holding portion 23a, which has a substantially circular cylindrical shape, and an arm portion 23b protruding in the radially outward direction (direction orthogonal to the optical axis O) in an arm-like manner from the side surface of the lens holding portion 23a.

The lens holding portion 23a is capable of holding the movable lens 11b inside. The lens holding portion 23a has an outer diameter allowing the lens holding portion 23a to be fitted in the circular cylindrical portion 21a of the fixed frame 21 with a predetermined gap, and is configured to be slidable in the direction of the optical axis O inside the circular cylindrical portion 21 a. The arm portion 23b is inserted through the slit 21b in a state where the lens holding portion 23a is fitted in the circular cylindrical portion 21a. Rotation of the movable lens holding frame 23 around the optical axis O is restricted by the arm portion 23b inserted in the slit 21b.

The arm portion 23b protrudes radially outward with respect to the circular cylindrical portion 21a in a state where the lens holding portion 23a is fitted in the circular cylindrical portion 21a. Specifically, the arm portion 23b has a length which causes the arm portion 23b to protrude radially outward up to a position overlapping the center of the through hole 21d provided to the holding portion 21c of the fixed frame 21.

The arm portion 23b is provided in such a manner as to abut the flat portion 22b of the pressing protrusion portion 22a of the object-side lens holding frame 22 before the lens holding portion 23a is abutted when the movable lens holding frame 23 is moved to the distal end side (the object side). Fig. 8 shows a state where the arm portion 23b abuts the flat portion 22b of the pressing protrusion portion 22a, and the movable lens holding frame 23 is at a position closest to the distal end side within the movable range.

On the other hand, when the movable lens holding frame 23 is moved to the proximal end side (the image side), the arm portion 23b is provided so as to abut a part, not shown, of the fixed frame 21 or a spacer, not shown, fixed to the fixed frame 21 before the lens holding portion 23a is abutted. In this manner, in the present embodiment, the movable range of the movable lens holding frame 23 in the direction of the optical axis O is set in the range up to a point where the arm portion 23b abuts a fixed part of the fixed frame 21.

The movable lens holding frame 23 moves in the distal end direction by the arm portion 23b being pressed by the pressing portion 32 disposed in the guide pipe 33 in a manner capable of moving forward and backward. The pressing portion 32 is disposed in a manner capable of moving forward and backward in the direction of the optical axis O along the guide pipe 33 inserted in the through hole 21d of the holding portion 21c.

A recessed portion 23c is provided on the surface, on the distal end side, of the arm portion 23b. A columnar core metal 25 is fitted in the recessed portion 23c in a manner protruding in a direction which is substantially parallel to the optical axis O. The core metal 25 is fixed to the arm portion 23c by an adhesive provided at a bottom portion of the recessed portion 23c. The core metal 25 is provided at a position protruding into the recessed portion 22c provided to the pressing protrusion portion 22a of the object-side lens holding frame 22. The core metal 25 is a part for preventing buckling of a first spring 31 constituting the drive mechanism section 30 described below.

Furthermore, the image-side lens holding frame 24 is fixed inside the circular cylindrical portion 21a of the fixed frame 21, on the proximal end side with respect to the movable lens holding frame 23 and on the object side with respect to the cover glass 13. The image-side lens holding frame 24 is a substantially circular cylindrical member for holding the fixed lens 11a, of the objective lens 11, positioned on the image side with respect to the movable lens 11b is.

On the proximal end side of the fixed frame 21, the image pickup device 12, the circuit board 14, and a distal end portion of the electrical cable 115 are surrounded by a cylindrical shield frame 15 made of a thin metal plate, and the inside of the shield frame 15 is filled with an electrically insulating sealing resin 16. Also, the shield frame 15 and the distal end portion of the electrical cable 115 are sheathed in a heat-shrinkable tube 17.

The movable lens holding frame 23 of the lens barrel section 20 described above is driven forward and backward in the direction of the optical axis O by the drive mechanism section 30 disposed at the side portion of lens barrel section 20. In the present embodiment, the drive mechanism section 30 is configured to drive the movable lens holding frame 23 in the direction of the optical axis O by expansion/contraction of the SMA wire 41. A configuration of the drive mechanism section 30 of the present embodiment will be described below.

As shown in Figs. 8 and 9, the drive mechanism section 30 is configured by including a driving force transmission portion 30a disposed at a side portion of the lens barrel section 20, a driving force generation portion 30b disposed on the proximal end side with respect to the driving force transmission portion 30a, and a coupling portion 30c connecting the driving force transmission portion 30a and the driving force generation portion 30b.

The driving force transmission portion 30a is configured by including the pressing protrusion portion 22a, the first spring 31, the pressing portion 32, the guide pipe 33, a second spring 34, a first outer tube 35, a first inner tube 36, and an inner wire 37.

The guide pipe 33 is a circular cylindrical pipe, and is fixed to the holding portion 21c in a state where the distal end portion of the guide pipe 33 is inserted in the through hole 21 d provided to the holding portion 21c of the fixed frame 21. The guide pipe 33 is positioned and fixed at a side portion of the fixed frame 21 by the holding portion 21c in such a way that a center axis is substantially parallel to the optical axis O.

The first outer tube 35 is connected to a proximal end of the guide pipe 33. The first outer tube 35 is a pipe of a synthetic resin such as polyether ether ketone resin (PEEK), and the first inner tube 36 is disposed inside. The first inner tube 36 is a pipe of a synthetic resin such as polytetrafluoroethylene resin (PTFE), and the inner wire 37 is inserted inside.

The first outer tube 35 and the first inner tube 36 are configured to be bendable according to bending of the bending portion 109 of the insertion section 102 of the endoscope 101, and to resist tension applied to the inner wire 37 inserted inside.

The piston-like pressing portion 32 is disposed in the guide pipe 33 in a manner capable of sliding in an axial direction. The pressing portion 32 protrudes from the distal end of the guide pipe 33 in the distal end direction, and abuts the arm portion 23b of the movable lens holding frame 23. A distal end of the inner wire 37 is fixed to the pressing portion 32.

Furthermore, the second spring 34 for biasing the pressing portion 32 in the distal end direction is disposed in the guide pipe 33. In the present embodiment, the second spring 34 is a compression coil spring. Accordingly, when tension is not applied to the inner wire 37, the pressing portion 32 presses the arm portion 23b of the movable lens holding frame 23 in the distal end direction by the biasing force of the second spring 34.

For its part, the first spring 31 is disposed to bias the movable lens holding frame 23 in the proximal end direction. In the present embodiment, the first spring 31 is a compression coil spring, and is disposed in the recessed portion 22c provided to the pressing protrusion portion 22a of the object-side lens holding frame 22. Accordingly, the first spring 31 is disposed opposite the pressing portion 32 across the arm portion 23b of the movable lens holding frame 23.

The first spring 31 is configured such that the force of biasing the arm portion 23b in the proximal end direction is weaker than the force, of the second spring 34, for biasing the arm portion 23b in the distal end direction. Accordingly, when tension is not applied to the inner wire 37, the arm portion 23b is moved in the distal end direction by the biasing force of the second spring 34, and is made to abut the pressing protrusion portion 22a. That is, when tension is not applied to the inner wire 37, the movable lens holding frame 23 is positioned at a distal end of the movable range.

Moreover, when tension is applied to the inner wire 37 and the second spring 34 is contracted, and the pressing portion 32 is moved in the proximal end direction, the arm portion 23b is moved to the proximal end side by the biasing force of the first spring 31. That is, the driving force transmission portion 30a is configured such that the pressing portion 32 moves forward and backward in the direction of the optical axis O according to a change in the tension on the inner wire 37, and such that the movable lens holding frame 23 is moved in the direction of the optical axis O by the biasing force of the first spring 31.

The driving force generation portion 30b is configured to generate tension to be applied to the inner wire 37. As shown in Fig. 9, the driving force generation portion 30b is configured by including an SMA wire 41, a second outer tube 42, a second inner tube 43, and an electric wire 44. In the present embodiment, the driving force generation portion 30b is provided closer to the proximal end side with respect to the bending portion 109 of the insertion section 102 of the endoscope 101 is, and is inserted in the flexible tube portion 108.

The second outer tube 42 is a pipe of a synthetic resin such as polyether ether ketone resin (PEEK), and the second inner tube 43 is disposed inside. The second inner tube 43 is a pipe of a synthetic resin such as polytetrafluoroethylene resin (PTFE), and the SMA wire 41 is inserted inside.

The second outer tube 42 and the second inner tube 43 correspond to the flexible portion 5 among the basic components of the signal line installation structure shown in Fig. 1, and are configured to be bendable according to bending of the flexible tube portion 108 of the insertion section 102 of the endoscope 101, and to resist tension generated by the SMA wire 41 inserted inside.

Distal ends of the second outer tube 42 and the second inner tube 43 are coupled to proximal ends of the first outer tube 35 and the first inner tube 36 by a coupling pipe 51 of the coupling portion 30c. The coupling pipe 51 corresponds to the rigid portion 4 among the basic components of the signal line installation structure shown in Fig. 1, and is formed by a tubular member of a metal material such as stainless steel.

The proximal end of the first outer tube 35 and a distal end of the coupling pipe 51 are fixed by an adhesive. A proximal end of the coupling pipe 51 is fixed to the proximal ends of the second outer tube 42 and the second inner tube 43 by crimping and flattening the coupling pipe 51.

The electric wire 44 corresponds to the signal line 6 among the basic components of the signal line installation structure shown in Fig. 1, and is electrically connected to the SMA wire 41 via the coupling pipe 51. Specifically, a distal end of the electric wire 44 is soldered and fixed to an outer circumferential surface of the coupling pipe 51, and a first electrical connection portion 44a corresponding to the electrical connection portion 6a of the signal line 6 is thus formed.

Moreover, the electric wire 44 is helically wound around an outer circumferential portion extending from the coupling pipe 51 to the second outer tube 42 with the first electrical connection portion 44a as a starting point, and a spiral portion 44b corresponding to the spiral portion 6b of the signal line 6 is provided across a predetermined length, and the signal line installation structure of the present invention is thus formed. A proximal end side on the rear of the spiral portion 44b is linearly inserted in the flexible tube portion 108, and extends to be connected to the endoscope connector 105.

Furthermore, the coupling pipe 51 is electrically connected to the SMA wire 41 via a wire coupling portion 52 disposed in the coupling pipe 51. The wire coupling portion 52 couples together a distal end of the SMA wire 41 and a proximal end of the inner wire 37, and also couples together the SMA wire 41 and a lead wire 45 which is electrically connected to the coupling pipe 51.

The wire coupling portion 52 includes a coupling tube 53 of a resin material, and a wire holding member 54, which is a metal tube and which is disposed in the coupling tube 53. An adhesive fills a space between the inner circumferential surface of the coupling tube 53 and the wire holding member 54, and the wire holding member 54 is fixed inside the coupling tube 53 by the adhesive.

The coupling tube 53 is formed of a synthetic resin such as polyether ether ketone resin (PEEK) in a manner capable of sliding in the coupling pipe 51 of a metal material. Also, the wire holding member 53 is a tubular member of metal, and by crimping the tubular member in a state where the inner wire 37, the SMA wire 41 and the lead wire 45 are inserted in the tubular member, the distal end of the SMA wire 41 and the proximal end of the inner wire 37 are coupled together, and also, the SMA wire 41 and the coupling pipe 51 are electrically connected by the lead wire 45.

One end of the lead wire 45 is soldered and fixed to a second electrical connection portion 45a provided on the outer circumferential surface on the distal end side of the coupling pipe 51, and the lead wire 45 is drawn from the second electrical connection portion 45a into the coupling pipe 51 through an opening section opened on the outer circumferential surface of the coupling pipe 51. The other end of the lead wire 45 drawn into the coupling pipe 51 is electrically connected to the SMA wire 41 via the wire holding member 53. The lead wire 45 is disposed in the coupling pipe 51 with some slack so as to be capable of following the movement of the distal end portion of the SMA wire 41 that moves forward or backward in the axial direction inside the coupling pipe 51.

Note that, although not shown, a proximal end of the SMA wire 41 is fixed so that the position in the longitudinal direction with respect to a proximal end of the second outer tube 42 is not changed. Also, a distal end of an electric wire, not shown, is electrically connected to a proximal end portion of the SMA wire 41. A proximal end of the electric wire that is electrically connected to the proximal end portion of the SMA wire 41 is provided at the endoscope connector 105, and the electric wire is capable of being electrically connected to the power source section 120a via the endoscope connector 105.

Furthermore, the outer circumference of a connection portion of the proximal end of the first outer tube 35 and the distal end of the coupling pipe 51 is sheathed in a first heat-shrinkable tube 56. An adhesive fills a space between the inner circumferential surface of a distal end portion of the first heat-shrinkable tube 56 and the outer circumferential surface of the first outer tube 35, and between the inner circumferential surface of a proximal end portion of the first heat-shrinkable tube 56 and the outer circumferential surface of the coupling pipe 51. According to such a configuration, water-tightness at the connection portion of the proximal end of the first outer tube 35 and the distal end of the coupling pipe 51 is realized.

Furthermore, the outer circumferential surface of the first outer tube 35 and the outer circumferential surface of the first heat-shrinkable tube 56 are sheathed in a second heat-shrinkable tube 59. A proximal end portion of the second heat-shrinkable tube 59 is fixed by an adhesive filling a space between the second heat-shrinkable tube 59 and the outer circumferential surface of the first heat-shrinkable tube 56. Moreover, on the proximal end side with respect to the second heat-shrinkable tube 59, the outer circumferential surface of the first heat-shrinkable tube 56 and the outer circumferential surface of the second outer tube 42 are sheathed in a third heat-shrinkable tube 60. A distal end portion of the third heat-shrinkable tube 60 is fixed at the proximal end portion of the second heat-shrinkable tube 59 by an adhesive 58 filling a space between the distal end portion of the third heat-shrinkable tube 60 and the outer circumferential surface of the first heat-shrinkable tube 56. Water-tightness of the coupling portion 30c is thereby reliably maintained by the second heat-shrinkable tube 59 and the third heat-shrinkable tube 60.

The third heat-shrinkable tube 60 corresponds to the outer skin tube 2 among the basic components of the signal line installation structure shown in Fig. 1, and the electric wire 44 corresponding to the signal line 6 is disposed between the inner circumferential surface of the third heat-shrinkable tube 60 and the outer circumferential surfaces of the coupling pipe 51 and the second outer tube 42.

At the driving force generation portion 30b described above, the SMA wire 41 is capable of being electrically connected to the power source section 120a via a pair of electric wires, and an electrical current that is outputted from the power source section 120a flows from the SMA wire 41 to the electric wire 44 via the lead wire 45 and the coupling pipe 51, and is returned to the ground from the electric wire 44. The SMA wire 41 generates heat according to the applied electrical current, and shrinks according to heat generation. When the SMA wire 41 shrinks, tension is applied to the inner wire 37 connected to the distal end of the SMA wire 41, and a driving force for driving the movable lens holding frame 23 is generated.

Normally, endoscopic observation performed by controlling application of an electrical current to the SMA wire 41 and moving the movable lens holding frame 23 forward and backward is observation that is performed while bending the bending portion 109 and orienting the distal end portion 110 in a desired direction. Accordingly, bending displacement is repeatedly caused at the electric wire 44 that is inserted in the flexible tube portion 108 from the first electrical connection portion 44a on the outer circumference of the coupling pipe 51 of the driving force generation portion 30b.

With respect to the repeated bending displacement, the electric wire 44 is disposed according to the installation structure according to which the electric wire 44 is helically wound around the outer circumferential surface across the coupling pipe 51 and the second outer tube 42, and thus, disconnection due to repeated bending stress is not caused.

That is, when the second outer tube 42 and the second inner tube 43 in the flexible tube portion 108 are bent from adjacent portions to the coupling pipe 51 according to a bending operation of the bending portion 109, the force applied to the spiral portion 44b of the electric wire 44 is made a force in the twisting direction in a predetermined range from the outer circumference of the coupling pipe 51 to the outer circumference of the second outer tube 42 where the bending load is the greatest, and disconnection of the electric wire 44 due to a force in the bending direction may be prevented.

With respect to the spiral portion 44b of the electric wire 44, a helical winding pitch p, the number of pitches n (the number of windings), and a winding distance L across an end portion of the coupling pipe 51 and the second outer tube 42 (substantially the length of the spiral portion 44b) are set taking into account the expected maximum bending diameter, the material and the wire diameter of the electric wire 44, and the like. For example, in the example of the endoscope 101 of the present embodiment, p ≤ 30 mm, n ≥ 7, and L ≥ 60 mm are set, and a force which may possibly cause breakage is prevented from being applied to the electric wire 44 itself even if a bending stress is repeatedly applied to the spiral portion 44b.

As described above, according to the present embodiment, a signal line installed between the outer circumferential surfaces of the rigid portion and the flexible portion of the elongated member and the inner surface of the outer skin tube is at least partially disposed according to the installation structure according to which the signal line is helically wound around the outer circumferences of the rigid portion and the flexible portion. According to the signal line installation structure, even if the elongated member is repeatedly bent, disconnection of the signal line due to the repeated bending stress may be reliably prevented and occurrence of inconveniences due to disconnection of the signal line may be prevented in advance, and thus, the mechanical and electrical reliability of parts may be increased.

The present application claims priority from Japanese Patent Application No. 2015-117714 filed in Japan on June 10, 2015, the entire contents of which are incorporated in the specification, Claims and drawings of this application by reference.

## Claims

1. A signal line installation structure of an elongated member including an outer skin tube that is cylindrically formed and that extends along a predetermined axis, an internal component that is housed inside the outer skin tube and that includes a rigid portion and a flexible portion that are disposed next to each other in a direction of the axis, and a signal line that is installed across the rigid portion and the flexible portion and between an inner circumferential surface of the outer skin tube and an outer circumferential surface of the internal component, wherein
the signal line that is disposed at outer circumferential portions of the rigid portion and the flexible portion is at least partially helically wound around outer circumferential surfaces of the rigid portion and the flexible portion.

2. The signal line installation structure of the elongated member according to claim 1, wherein the signal line is helically wound around at least a part of the flexible portion that is adjacent to the rigid portion.

3. The signal line installation structure of the elongated member according to claim 1 or 2, wherein the signal line is also helically wound around a part of the rigid portion that is adjacent to the flexible portion.

4. The signal line installation structure of the elongated member according to claim 1, wherein the flexible portion is formed of resin.

5. The signal line installation structure of the elongated member according to claim 1, wherein the rigid portion is formed of metal.

6. The signal line installation structure of the elongated member according to claim 5, wherein the outer circumferential surface of the rigid portion and the signal line are electrically connected to each other.

7. The signal line installation structure of the elongated member according to claim 6, wherein the outer circumferential surface of the rigid portion and the signal line are electrically connected to each other by soldering.

8. The signal line installation structure of the elongated member according to claim 1, wherein the outer skin tube is disposed at an insertion section of an endoscope that is inserted into a subject.
